# EUROPÄISCHE PATENTANMELDUNG

(11) **EP 2 628 469 A2**
(43) Veröffentlichungstag der Anmeldung: **21.08.2013**
(21) Anmeldenummer: 12196259.1
(22) Anmeldetag: 10.12.2012
(51) Int. Cl.: A61F 2/966

(54) **Crimpvorrichtung und Verfahren zur Montage eines Vaskularstents**

(30) Priorität: 16.02.2012 US 201261599431 P
(71) Anmelder: Biotronik AG, 8180 Bülach (CH)
(72) Erfinder: Fargahi, Amir, 8180 Bülach (CH)
(74) Vertreter: Lindner-Vogt, Karin L.

(57) **Zusammenfassung**

Crimpvorrichtung zur Montage eines Vaskularstents oder einer in einen Vaskularstent eingefügten Herzklappenprothese auf einen Einführkatheter, umfassend ein Formwerkzeug, das eine rotationssymmetrische Formkavität hat, welche an einem Ende einen Stent-Aufnahmeabschnitt, am gegenüberliegenden Ende einen Stent-Ausgabeabschnitt und zwischen diesen einen Verjüngungs-Abschnitt umfasst, und ein Stoßwerkzeug, das einen Halte- und Handhabungsabschnitt und einen Stent-Formungsabschnitt umfasst, wobei letzterer zum Eindringen in die Formkavität des Formwerkzeugs vom offenen Ende des Stent-Aufnahmeabschnitts und zum Erfassen und Zurückstoßen des dort eingesetzten Vaskularstents durch den Verjüngungs-Abschnitt ausgebildet ist.

## Beschreibung

Die Erfindung betrifft eine Crimpvorrichtung zur Montage eines Vaskularstents oder einer in einen Vaskularstent eingefügten Herzklappenprothese auf einen Einführkatheter. Sie betrifft des Weiteren ein Verfahren zur Montage eines Vaskularstents oder einer in einen Vaskularstent eingefügten Herzklappenprothese auf einen Einführkatheter.

Vaskularstents (Gefäßstützen), die in geschädigte Gefäße mittels eines Einführkatheters eingeführt werden, um dauerhaft einen Blutdurchfluss zu sichern, sind seit langem bekannt und massenhaft im klinischen Einsatz. Bekanntermaßen werden Vaskularstents in einem Zustand reduzierten Durchmessers auf den Einführkatheter montiert und mit diesem eingeführt und nehmen im implantierten Zustand, bewirkt beispielsweise durch die Dilatation eines in ihnen liegenden inflatierten Ballons oder durch Gedächtniseffekte, im Körper des Patienten ihren expandierten Gebrauchszustand ein.

In jüngerer Zeit sind Herzklappenprothesen bekannt geworden, die minimal invasiv implantierbar sind und bei denen die eigentliche Herzklappe in einem Vaskularstent angebracht ist, der die oben erwähnten typischen Einsatzmerkmale hat. Bei derartigen Herzklappenprothesen, etwa den Produkten "Corevalve" der Firma Medtronic Inc. oder "Sapien" der Firma Edwards Life Sciences Inc., erfordert die Montage auf den Einführkatheter spezielle Werkzeuge und Handhabungen. Die bekannten Werkzeuge sind relativ kompliziert aufgebaut und zu handhaben, womit die Montage der Prothese auf den Einführkatheter zu einem relativ umständlichen und auf kostspieligen Vorgang wird.

Es ist daher Aufgabe der Erfindung, eine verbesserte Crimpvorrichtung und ein verbessertes Verfahren zur Montage eines Vaskularstents oder einer in einen Vaskularstent eingefügten Herzklappenprothese auf einen Einführkatheter bereitzustellen, die insbesondere unkompliziert und kostengünstig sind.

Diese Aufgabe wird in ihrem Vorrichtungsaspekt durch eine Crimpvorrichtung mit den Merkmalen des Anspruchs 1 und in ihrem Verfahrensaspekt durch ein Verfahren mit den Merkmalen des Anspruchs 9 gelöst. Zweckmäßige Fortbildungen des Erfindungsgedankens sind Gegenstand der abhängigen Ansprüche.

Die Erfindung schließt die Überlegung ein, die Montage der Prothese auf den Einführkatheter, der üblicherweise einen Innenschlauch und einem diesen gegenüber verschieblichen Außenschlauch umfasst, in einem zusammenhängenden Arbeitsgang des Reduzierens des Prothesen-Durchmessers und des Einschiebens zwischen Innen- und Außenschlauch des Einführkatheters auszuführen. Sie schließt weiter den Gedanken ein, hierfür eine Vorrichtung bereitzustellen, die die Verringerung des Prothesendurchmessers durch ihre geometrische Konfiguration selbsttätig in einem einzigen einfachen Bedien-Schritt leistet. Des Weiteren schließt sie die Überlegung ein, zur Ausführung dieses Bedienschritts ein spezielles Werkzeug bereitzustellen, welches auf die durchmesser-verringernde geometrische Konfiguration der Vorrichtung abgestimmt ist. Des Weiteren setzt die angestrebte einfache Bedienung voraus, dass sowohl die Prothese als auch derjenige Abschnitt des Einführkatheters, wo sie eingesetzt werden soll, in der Vorrichtung vorab fixiert werden bzw. (unter Vorrichtungsaspekten) dass die Vorrichtung zu einer solchen Fixierung ausgebildet ist.

Nach obigem umfasst die Vorrichtung einerseits ein Formwerkzeug, das eine rotationssymmetrische Formkavität hat, welche an einem Ende einen Stent-Aufnahmeabschnitt, am gegenüberliegenden Ende einen Außenschlauch-Aufnahmeabschnitt und zwischen diesen einen Verjüngungs-Abschnitt umfasst. Des Weiteren umfasst sie ein Stoßwerkzeug, das einen Halte- und Handhabungsabschnitt und einen Stent-Formungsabschnitt umfasst, wobei letzterer zum Eindringen in die Formkavität des Formwerkzeugs vom offenen Ende des Stent-Aufnahmeabschnitts und zum Erfassen und Zurückstoßen des dort eingesetzten Vaskularstents durch den Verjüngungs-Abschnitt ausgebildet ist.

In einer Ausführung der Erfindung umfasst der Stent-Formungsabschnitt des Stoßwerkzeugs eine Mehrzahl von biegsamen Stäben oder sich zu ihrem freien Ende hin verjüngenden Lamellen und der Halte- und Handhabungsabschnitt eine kreisring- oder scheibenförmige Halterung, an deren Umfang die Stäbe, insbesondere in gleichen Winkelabständen, einseitig gehaltert sind. Die Anzahl der Stäbe bzw. Lamellen sollte hinreichend groß sein, um ein über den Umfang gleichmäßiges Hindurchschieben der Prothese durch den Verjüngungs-Abschnitt des Formwerkzeugs zu gewährleisten. Hierfür können drei Stäbe bzw. Lamellen ausreichend sein; aus derzeitiger Sicht bevorzugt ist aber eine Ausführung mit vier oder mehr Stäben bzw. Lamellen.

In einer weiteren Ausführung ist das Formwerkzeug mehrteilig, insbesondere zweiteilig und aufklappbar, ausgeführt. Grundsätzlich kann das Formwerkzeug auch einteilig ausgeführt oder aber aus drei oder mehr Teilen zusammengesetzt sein, wobei die konkrete Ausführung in Abhängigkeit von der speziellen geometrischen Konfiguration des Stents bzw. der Herzklappen-Prothese und von Kostenüberlegungen gewählt werden wird. Eine zweiteilige Ausführung erscheint aus derzeitiger Sicht als guter Kompromiss zwischen hohem Gebrauchswert und vertretbaren Kosten; und eine aufklappbare Konstruktion ist besonders leicht und zuverlässig zu bedienen.

In einer weiteren Ausführung ist vorgesehen, dass der Außenschlauch-Aufnahmeabschnitt des Formwerkzeugs einen größeren Durchmesser als das benachbarte Ende des Verjüngungs-Abschnitts hat, derart, dass das Ende des Außenschlauch-Aufnahmeabschnitts einen ringförmigen Anschlag für das distale Ende des Außenschlauchs des Einführkatheters bildet.

In einer weiteren Ausgestaltung ist der Verjüngungs-Abschnitt des Formwerkzeugs kegelstumpfförmig ausgebildet. In Abwandlung dieser Ausführung, die insbesondere für speziell geformte Stents vorteilhaft ist, ist der Verjüngungs-Abschnitt des Formwerkzeugs aus zwei kegelstumpfförmigen Abschnitten mit unterschiedlichem Kegelwinkel gebildet.

Des Weiteren kann die Wandung des Verjüngungs-Abschnitt des Formwerkzeugs gekrümmt sein. Mit letzterer Ausführung lassen sich spezielle Verformungsprofile oder Gebrauchs-Konfigurationen sehr gezielt herstellen.

In einer weiteren Ausführung ist das Formwerkzeug mindestens abschnittsweise aus durchsichtigem Material hergestellt. Diese Ausführung ermöglicht in vorteilhafter Weise eine Sichtkontrolle des Montage- bzw. Crimpprozesses ohne zwischenzeitliches Öffnen des Werkzeugs und die damit verbundenen Nachteile. Als Material kommt hierbei Plexiglas oder ein anderer hochtransparenter Kunststoff in Betracht; im Übrigen kann das Formwerkzeug auch aus Kunststoffen wie Polycarbonat oder Grilamid TR 55 hergestellt sein. Diese Werkstoffe eignen sich grundsätzlich auch zur Herstellung des Stoßwerkzeugs, für dessen Stäbe bzw. Lamellen aber auch Metalle (beispielsweise Edelstahl) eingesetzt werden können.

Um die Reibung zw. dem Formwerkzeug und dem Stosswerkzeug reduzieren, werden in einer weiteren Ausgestaltung die Kontaktoberflächen hydrophob oder hydrophil beschichtet.

Zweckmäßige Ausgestaltungen des Verfahrens ergeben sich im Wesentlichen unmittelbar aus oben erwähnten Vorrichtungsaspekten, so dass sich eine Wiederholung hier erübrigt.

Vorteile und Zweckmäßigkeiten der Erfindung werden im Übrigen aus der nachfolgenden Beschreibung von Ausführungsbeispielen anhand der Figuren deutlich. Es zeigen:
- Fig. 1A und 1B: perspektivische Darstellungen des Formwerkzeugs einer Ausführungsform der erfindungsgemäßen Vorrichtung, im halb geöffneten bzw. geschlossenen Zustand,
- Fig. 2: eine schematische Darstellung einer Ausführungsform des Stoßwerkzeugs und
- Fig. 3A bis 3D: schematische Darstellungen einer Ausführungsform der erfindungsgemäßen Vorrichtung in verschiedenen Phasen eines Montagevorgangs.

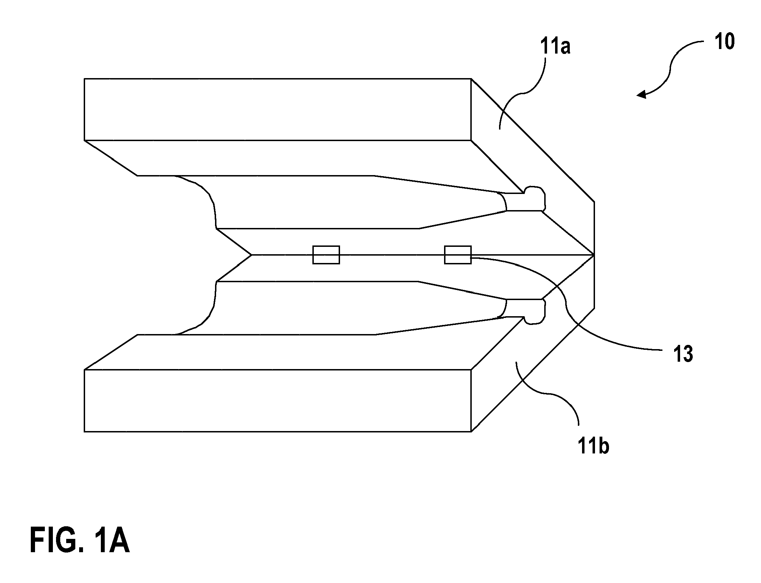

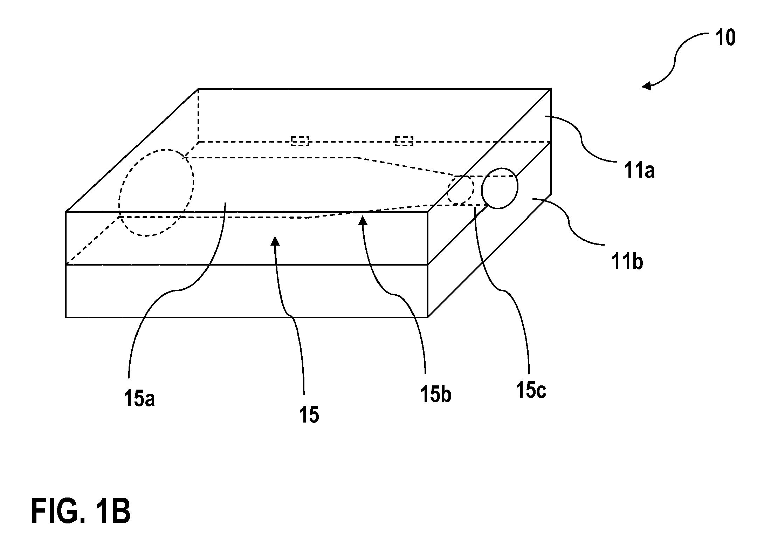

Fig. 1A und 1B zeigen ein Formwerkzeug 10 einer erfindungsgemäßen Crimpvorrichtung, das aus zwei gleichen Halbschalen 11a, 11b mit Klappscharnieren 13 aufgebaut ist, im halb aufgeklappten Zustand (Fig. 1A) bzw. im geschlossenen Zustand (Fig. 1B). Identische Ausformungen in beiden Halbschalen 11a, 11b bilden im zusammengeklappten Gebrauchszustand eine Formkavität 15, die einen Stent-Aufnahmeabschnitt 15a, einen Verjüngungs-Abschnitt 15b und einen Außenschlauch-Aufnahmeabschnitt 15c umfasst. Die Funktion der einzelnen Abschnitte der Formkavität wird aus Erläuterungen einer modifizierten Ausführung weiter unten deutlich.

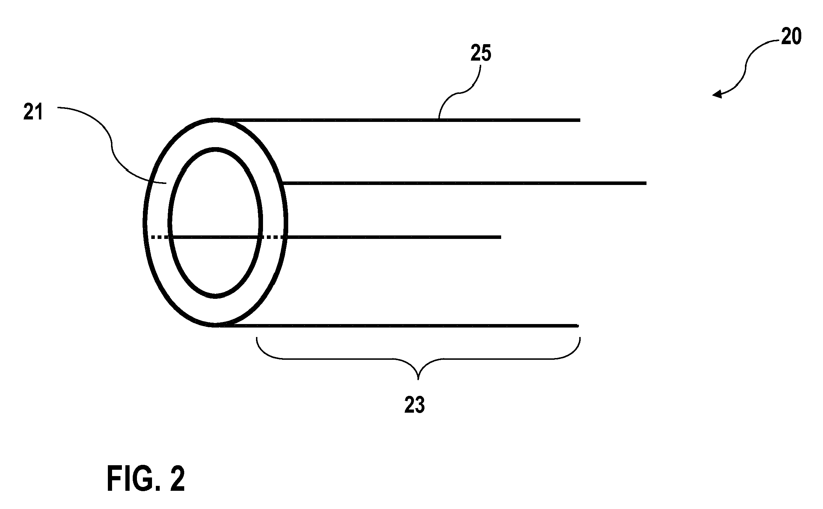

Fig. 2 zeigt schematisch den Aufbau eines Stoßwerkzeuges 20, welches zu einer erfindungsgemäßen Crimpvorrichtung gehört. Es umfasst einen als Kreisring ausgebildeten Halte- und Handhabungsabschnitt 21 und einen Stent-Formungsabschnitt 23, der durch vier in gleichen Winkelabständen einseitig am Halte- und Handhabungsabschnitt 21 befestigte biegsame Stäbe 25 gebildet ist.

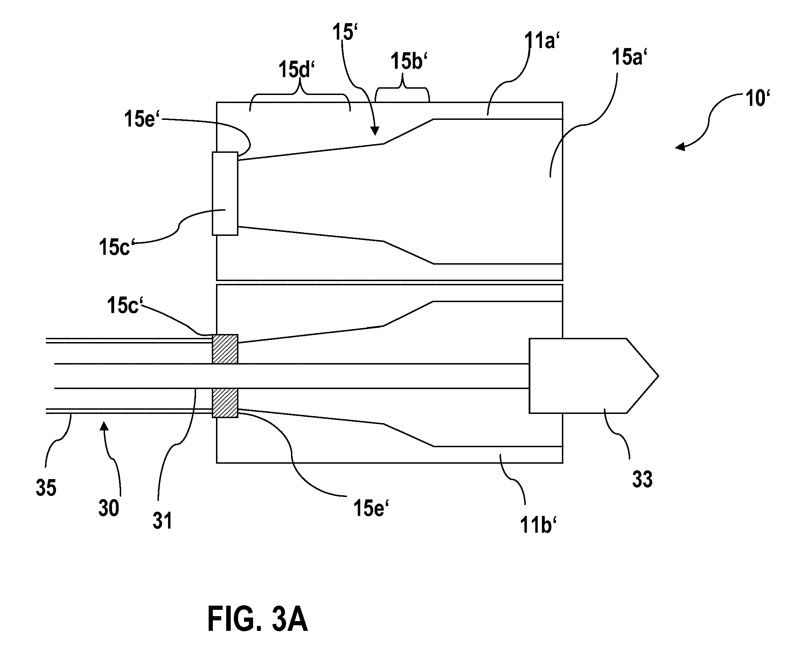

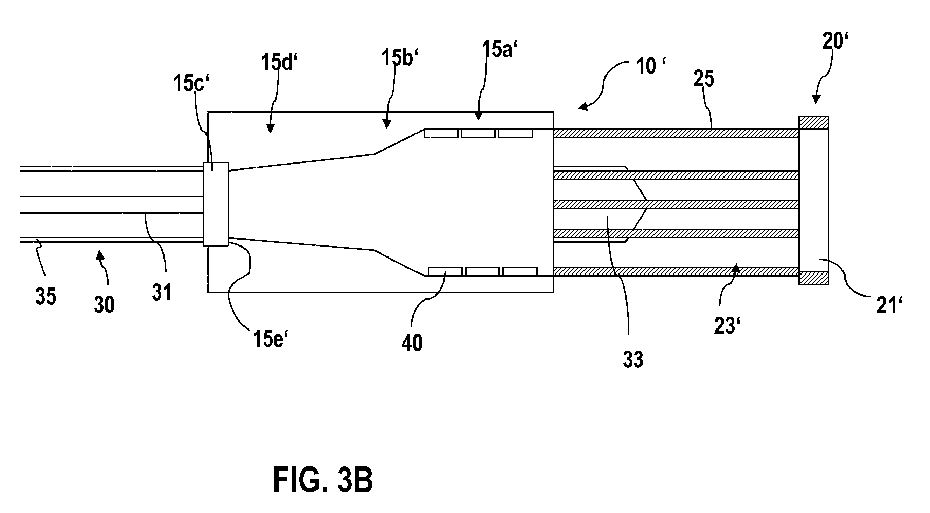

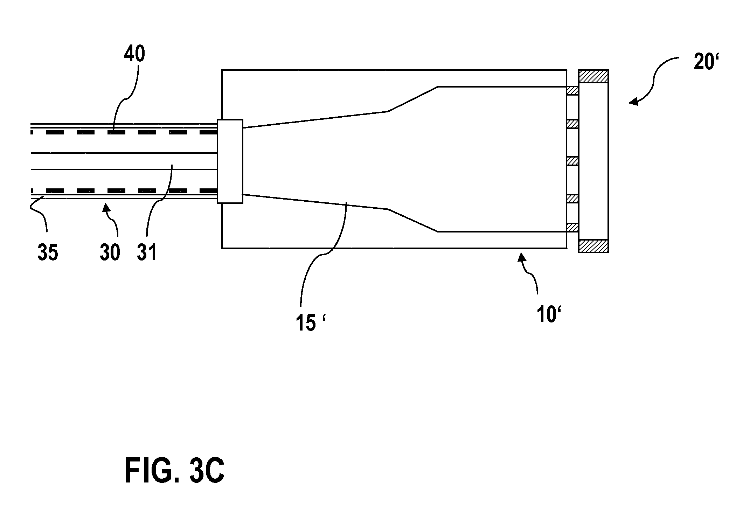

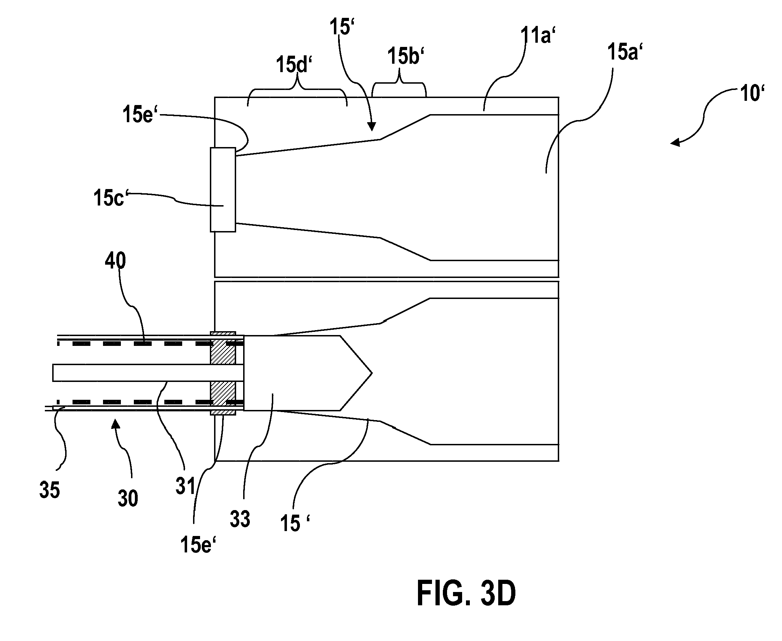

Fig. 3A bis 3D zeigen in schematischen Darstellungen eine gegenüber der Ausführung nach Fig. 1 und 2 modifizierte Crimpvorrichtung im Einsatz, und zwar in Fig. 3A im geöffneten Zustand mit eingesetztem Einführkatheter, in Fig. 3B im geschlossenen Zustand mit an das Formwerkzeug angesetztem Stoßwerkzeug, in Fig. 3C nach Abschluss des Montagevorganges, und in Fig. 3D im wieder aufgeklappten Zustand zum Entnehmen des fertigen Produkts. Es wird ausdrücklich darauf hingewiesen, dass nicht in allen Figuren alle Teile des Einführkatheters zu erkennen sind und die Darstellungen des Stoßwerkzeugs und des Stents (speziell was deren Länge angeht) in Fig. 3B und 3C nicht völlig konsistent miteinander sind. In Fig. 3B und 3C ist, obgleich diese Figuren das Formwerkzeug im geschlossenen Zustand zeigen, die Formkavität in gleicher Weise wie beim offenen Werkzeug dargestellt.

Das modifizierte Formwerkzeug 10' ist wieder aufklappbar aus zwei Halbschalen 11a', 11b' gebildet und hat eine Formkavität 15' mit einem Stent-Aufnahmeabschnitt 15a' und einem Außenschlauch-Aufnahmeabschnitt 15c'. Ihr Verjüngungs-Abschnitt ist aus zwei Kegelstumpf-Abschnitten 15b' und 15d' gebildet. Das modifizierte Stoßwerkzeug 20' umfasst neben dem ringförmigen Halte- und Handhabungsabschnitt 21' einen Stent-Formungsabschnitt 23', der eine größere Anzahl von Metallstäben 25 als die erste Ausführungsform umfasst.

In das Formwerkzeug 10' wird ein Einführkatheter 30 eingesetzt, der einen Innenschlauch 31 mit einer Spitze 33 und einem diesen gegenüber axial verschieblichen Außenschlauch 35 umfasst. Der Einführkatheter 30 wird derart in das Formwerkzeug eingelegt, dass das distale Ende des Außenschlauches 35 in den Außenschlauch-Aufnahmeabschnitt 15c' zu liegen kommt. Da der Durchmesser dieses Abschnitts größer als der Durchmesser des Verjüngungs-Abschnitts 15b', 15d' an dessen Ende ist, ist dort ein ringförmiger Anschlag 15e' gebildet, gegen den das distale Ende des Außenschlauches 35 stößt. Der Innenschlauch 31 mit der Spitze 33 wird distal aus dem derart fixierten Außenschlauch herausgezogen, so dass der Ringraum zwischen Innen- und Außenschlauch 31, 35 zugänglich wird.

Fig. 3B zeigt, wie in den Stent-Aufnahmeabschnitt 15a' ein Vaskularstent 40 im expandierten Zustand eingesetzt ist. Vom offenen Ende des Stent-Aufnahmeabschnitts 15a' her wird das Stoßwerkzeug 20' angesetzt und zum gegenüberliegenden Ende des Formwerkzeugs 10', also in Richtung auf das distale Ende des Außenschlauchs 35 des Einführkatheters 30, gedrückt. Die Enden der Stäbe 25 des Stoßwerkzeugs 20' greifen dabei in die am weitesten proximal liegenden (nicht dargestellten) Stege des Vaskularstents 40 ein und nehmen diese (und somit den ganzen Stent, unter Verformung) in Richtung der Stoßbewegung mit. Dabei wird der Stent durch den zweiteiligen Verjüngungs-Abschnitt 15b', 15d' hindurchgedrückt, wobei sein Durchmesser bis auf das für den Implantationsvorgang vorgesehen Endmaß reduziert wird. Zugleich wird der Stent in den Ringraum zwischen dem Innen- und Außenschlauch des Einführkatheters 30 hineingedrückt. Der Stent 40 nimmt letztlich die in Fig. 3C gezeigte Position im Einführkatheter ein. Dieses ist dann im gebrauchsfertigen Zustand. Fig. 3D zeigt das Formwerkzeug 10' im wieder geöffneten Zustand zum Entnehmen des Einführkatheters mit eingefügtem Stent.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Crimpvorrichtung zur Montage eines Vaskularstents (40) oder einer in einen Vaskularstent eingefügten Herzklappenprothese auf einen Einführkatheter (30), umfassend ein Formwerkzeug (10; 10'), das eine rotationssymmetrische Formkavität (15; 15') hat, welche an einem Ende einen Stent-Aufnahmeabschnitt (15a; 15a'), am gegenüberliegenden Ende einen Stent-Ausgabeabschnitt (15c; 15c') und zwischen diesen einen Verjüngungs-Abschnitt (15b; 15b', 15d') umfasst, und
ein Stoßwerkzeug (20; 20'), das einen Halte- und Handhabungsabschnitt (21; 21') und einen Stent-Formungsabschnitt (23; 23') umfasst, wobei letzterer zum Eindringen in die Formkavität des Formwerkzeugs vom offenen Ende des Stent-Aufnahmeabschnitts und zum Erfassen und Zurückstoßen des dort eingesetzten Vaskularstents durch den Verjüngungs-Abschnitt ausgebildet ist.

2. Crimpvorrichtung nach Anspruch 1, wobei der Stent-Formungsabschnitt (23; 23') des Stoßwerkzeugs (20; 20') eine Mehrzahl von biegsamen Stäben (25) oder sich zu ihrem freien Ende hin verjüngenden Lamellen und der Halte- und Handhabungsabschnitt (21; 21') eine kreisring- oder scheibenförmige Halterung umfasst, an deren Umfang die Stäbe, insbesondere in gleichen Winkelabständen, einseitig gehaltert sind.

3. Crimpvorrichtung nach Anspruch 1 oder 2, wobei das Formwerkzeug (10; 10') mehrteilig, insbesondere zweiteilig und aufklappbar, ausgeführt ist.

4. Crimpvorrichtung nach einem der vorangehenden Ansprüche, wobei der Stent-Ausgabeabschnitt zugleich als Außenschlauch-Aufnahmeabschnitt zum Festhalten eines Außenschlauchs des Einführkatheters ausgebildet ist.

5. Crimpvorrichtung nach Anspruch 4, wobei der Außenschlauch-Aufnahmeabschnitt (15c; 15c') des Formwerkzeugs (10; 10') einen größeren Durchmesser als das benachbarte Ende des Verjüngungs-Abschnitts (15b; 15b') hat, derart, dass das Ende des Außenschlauch-Aufnahmeabschnitts einen ringförmigen Anschlag (15e') für das distale Ende des Außenschlauchs (35) des Einführkatheters (30) bildet.

6. Crimpvorrichtung nach einem der vorangehenden Ansprüche, wobei der Verjüngungs-Abschnitt (15b; 15b') des Formwerkzeugs (10; 10') kegelstumpfförmig ausgebildet ist.

7. Crimpwerkzeug nach einem der Ansprüche 1 bis 5, wobei der Verjüngungs-Abschnitt des Formwerkzeugs (10; 10') aus zwei kegelstumpfförmigen Abschnitten (15b', 15d') mit unterschiedlichem Kegelwinkel gebildet ist.

8. Crimpvorrichtung nach einem der Ansprüche 1 bis 5, wobei die Wandung des Verjüngungs-Abschnitt des Formwerkzeugs gekrümmt ist.

9. Crimpvorrichtung nach einem der vorangehenden Ansprüche, wobei das Formwerkzeug (10; 10') mindestens abschnittsweise aus durchsichtigem Material hergestellt ist.

10. Crimpvorrichtung nach einem der vorangehenden Ansprüche, wobei die Kontaktflächen des Formwerkzeugs hydrophob oder hydrophil beschichtet sind.

11. Verfahren zur Montage eines Vaskularstents oder einer in einen Vaskularstent eingefügten Herzklappenprothese auf einen Einführkatheter, umfassend:
- Bereitstellen eines expandierten Vaskularstents (40) oder einer Herzklappenprothese mit expandiertem Vaskularstent
- Bereitstellen eines Einführkatheters (30) mit einem Innenschlauch (31) mit einer Katheterspitze (33) und einem gegenüber dem Innenschlauch verschieblichen Außenschlauch (35),
- Bereitstellen eines Formwerkzeugs (10; 10'), das eine rotationssymmetrische Formkavität (15; 15') hat, welche an einem Ende einen Stent-Aufnahmeabschnitt (15a; 15a'), am gegenüberliegenden Ende einen Außenschlauch-Aufnahmeabschnitt (15c; 15c') und zwischen diesen einen Verjüngungs-Abschnitt (15b; 15b', 15d') umfasst,
- Bereitstellen eines Stoßwerkzeugs (20; 20'), das einen Halte- und Handhabungsabschnitt (21; 21') und einen Stent-Formungsabschnitt (23; 23') umfasst, wobei letzterer zum Eindringen in die Formkavität des Formwerkzeugs vom offenen Ende des Stent-Aufnahmeabschnitts und zum Erfassen und Zurückstoßen des dort eingesetzten Vaskularstents durch den Verjüngungs-Abschnitt ausgebildet ist,
- Einsetzen des Vaskularstents oder der Herzklappenprothese in den Stent-Aufnahmeabschnitt des Formwerkzeugs,
- Einsetzen des distalen Endes des Einführkatheters in das Formwerkzeug, wobei das distale Ende des Außenschlauchs in dessen zurückgezogenem Zustand in dem Außenschlauch-Aufnahmeabschnitt des Formwerkzeugs fixiert wird,
- Einführen des Stent-Formungsabschnitts des Stoßwerkzeugs in das offene Ende des Stent-Aufnahmeabschnitt des Formwerkzeugs und Zurückstoßen des Vaskularstents oder der Herzklappenprothese durch die Formkavität unter Reduzierung des Durchmessers, mit gleichzeitigem Einschieben zwischen den Außenschlauch und Innenschlauch des Einführkatheters.
